Europäisches Patentamt

European Patent Office

Office européen des brevets

(11) Numéro de publication: **0 363 229**
**A1**

## DEMANDE DE BREVET EUROPEEN

(21) Numéro de dépôt: 89401329.1

(22) Date de dépôt: 12.05.89

(51) Int. Cl.⁵ **A61B 5/02**

(30) Priorité: 25.05.88 FR 8806924

(43) Date de publication de la demande:
11.04.90 Bulletin 90/15

(84) Etats contractants désignés:
**AT BE CH DE ES GB GR IT LI LU NL SE**

(71) Demandeur: SPENGLER S.A.
73-75 Avenue Laplace B.P. N 20
F-94114 Arcueil(FR)

(72) Inventeur: Aubrun, Bernard
14, Avenue Chinault
F-36100 Issoudun(FR)
Inventeur: Mitelmann, Pierre-Henri
17bis, Avenue de Wailly
F-78290 Croissy S/Seine(FR)

(74) Mandataire: Brycman, Jean et al
CABINET ORES 6, Avenue de Messine
F-75008 Paris(FR)

(54) **Sphygmomanomètre.**

(57) Sphygmomanomètre comprenant, réunis en un ensemble unitaire, une poire (12) de mise en pression d'un brassard de mesure, un manomètre (14) de lecture proprement dit de la pression artérielle, une valve ou soupape (28) de mise à l'air libre du brassard et des moyens de commande pour l'ouverture progressive de ladite valve ou soupape pour réduire graduellement la pression du brassard gonflé en vue de la détermination des pressions systolique et diastolique, d'une part, et de maintien de la valve (28) dans sa condition de pleine ouverture pour la mise à l'air libre complète et rapide du brassard, d'autre part.

Les moyens de commande sont réalisés sous forme d'un bouton (17) monté à coulissement et auquel est associé un mécanisme de poussoir à dentures (51, 60, 65) organisé de manière telle que le blocage de ladite valve (28) dans sa condition de pleine ouverture pour la mise à l'air libre rapide du brassard est obtenu automatiquement après une course d'enfoncement prédéterminée du bouton-poussoir.

FIG.2

# SPHYGMOMANOMETRE

L'invention a pour objet un sphygmomanomètre du type de ceux réunissant en un ensemble unitaire la poire en caoutchouc usuelle de mise en pression du brassard de mesure, le manomètre de lecture proprement dit de la pression artérielle d'un patient en cours d'examen et les moyens d'actionnement de la valve ou soupape servant à progressivement réduire la pression de gonflage du brassard pour la détermination de la pression systolique et diastolique.

Pour faciliter leur utilisation, aussi bien en ce que concerne l'actionnement progressif de la valve de décharge que la mise rapide à l'atmosphère du brassard en fin de mesure, on a déjà proposé, par exemple dans FR-A-2 239 230, de munir des appareils de ce type d'une touche basculante agissant contre la force d'un ressort, un dispositif d'encliquetage rendu opératoire en fin de manoeuvre d'actionnement empêchant le retour de la touche pour la mise rapide à l'air libre du brassard de mesure. Bien que le système à touche basculante soit satisfaisant, il est apparu à la Demanderesse que des sphygmomanomètres de ce type pouvaient encore être perfectionnés pour rendre leur commande plus ergonomique que celle des dispositifs connus et, de ce fait, faciliter encore davantage leur emploi.

C'est, par conséquent, un but de l'invention de fournir un sphygmomanomètre du type de ceux décrits ci-dessus dans lequel la commande par l'utilisateur à l'aide d'un seul doigt, par exemple le pouce, soit rendue aussi simple que possible.

Un sphygmomanomètre selon l'invention, comprenant réunis en un ensemble unitaire une poire de mise en pression d'un brassard de mesure, un manomètre de lecture proprement dit de la pression artérielle, une valve ou soupape de mise à l'air libre du brassard et des moyens de commande pour l'ouverture progressive de ladite valve ou soupape pour réduire graduellement la pression du brassard gonflé en vue de la détermination des pressions systolique et diastolique, d'une part, et de maintien de la valve dans sa condition de pleine ouverture pour la mise à l'air libre complète et rapide du brassard, d'autre part, est caractérisé en ce que lesdits moyens de commande sont réalisés sous forme d'un bouton monté à coulissement et auquel est associé un mécanisme de poussoir à dentures organisé de manière telle que le blocage de ladite valve dans sa condition de pleine ouverture pour la mise à l'air libre rapide du brassard est obtenu automatiquement après une course d'enfoncement prédéterminée du bouton-poussoir, un nouvel appui sur ce dernier provoquant le déblocage de la valve et sa fermeture sous l'action de moyens élastiques préalablement mis sous tension ainsi que le retour du bouton-poussoir dans sa condition initiale pour une nouvelle mesure de pression.

Dans une forme de réalisation préférée, le mécanisme de poussoir à dentures associé au bouton est du type à rampes frontales et est disposé dans le corps du sphygmomanomètre entre le bouton-poussoir et l'organe mobile de la valve ou soupape.

Cette dernière est avantageusement constituée par un siège fixe ménagé par l'alésage axial tronconique d'un manchon dans lequel est monté à coulissement l'organe mobile conformé suivant une tige présentant une portée tronconique sur une partie de sa longueur, entre une queue de centrage des moyens élastiques de rappel dudit organe et une tête d'appui du bouton-poussoir de manoeuvre.

D'autres caractéristiques et avantages de l'invention apparaîtront de la description qui suit, faite à titre d'exemple et en référence au dessin annexé dans lequel:

- la figure 1 est une vue en élévation d'un sphygmomanomètre selon l'invention ;
- la figure 2 est une vue en partielle en coupe longitudinale selon la ligne 2-2 de la figure 1, certaines parties du mécanisme d'actionnement étant représentées sous forme schématique ;
- la figure 3 est une vue partielle en coupe longitudinale et à plus grande échelle du mécanisme d'actionnement dans une première condition ;
- la figure 4 est une vue analogue à celle de la figure 3 pour une autre condition du mécanisme d'actionnement ;
- la figure 5 est une vue en coupe longitudinale d'une partie constitutive du mécanisme ;
- la figure 6 est une vue en coupe selon la ligne 6-6 de la figure 5 ;
- la figure 7 est une vue en coupe longitudinale d'une autre partie constitutive du mécanisme ;
- la figure 8 est une vue en coupe selon la ligne 8-8 de la figure 7 ;
- la figure 9 est une vue en coupe longitudinale d'encore une autre partie constitutive du mécanisme ;
- la figure 10 est une vue en coupe selon la ligne 10-10 de la figure 9 ;
- la figure 11 est une vue éclatée, à plus grande échelle, de la valve ou soupage du sphygmomanomètre selon l'invention.

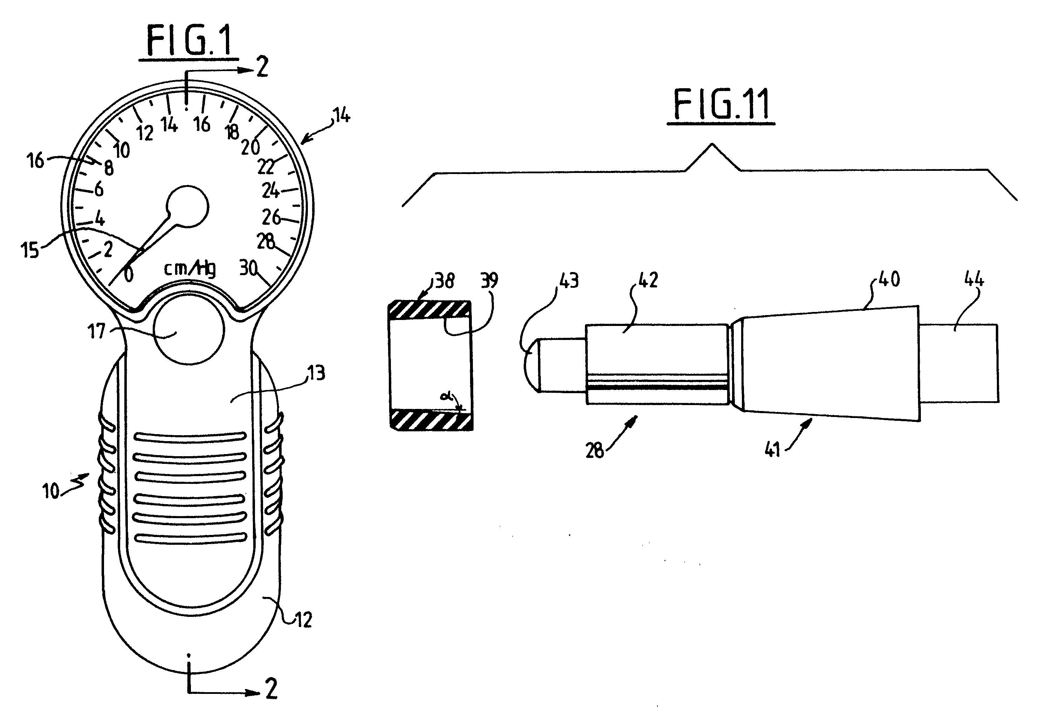

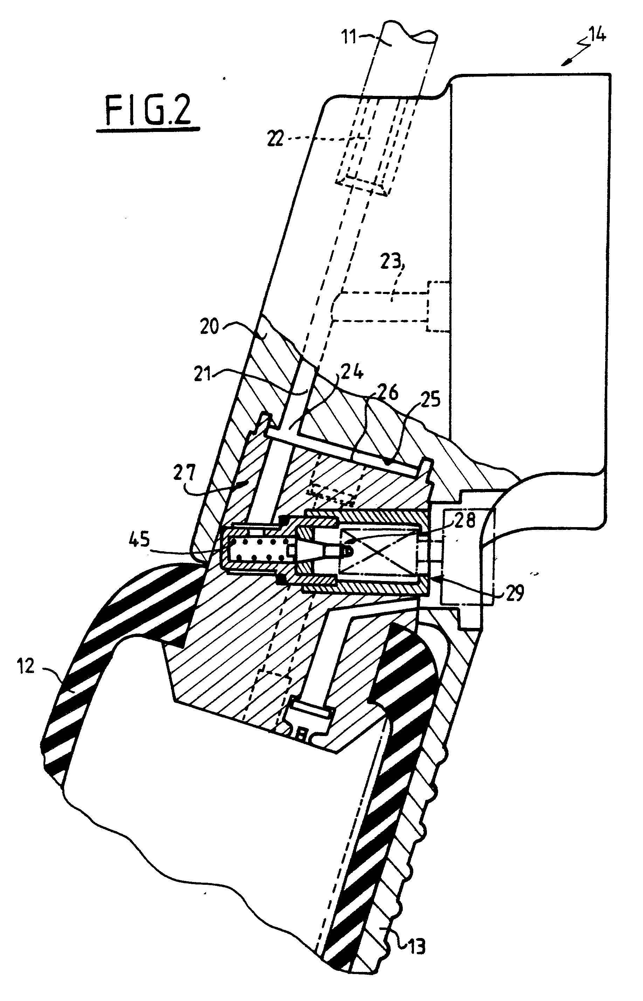

Un sphygmomanomètre 10, figure 1, du type auquel s'applique l'invention, comprend un brassard gonflable, no représenté, relié par une tubulure souple 11, figure 2, à un ensemble unitaire réunissant une poire 12 de gonflage du brassard, une cuiller 13 rigide recouvrant en partie ladite poire sur sa face antérieure frontale, un manomètre proprement dir 14 dont l'aiguille 15 coopère avec une graduation 16 d'un cadran de lecture et, sur la face avant de la cuiller 13, un organe 17 de commande d'une valve ou soupape de mise à l'air libre progressivement d'abord, puis rapidement ensuite, du brassard après qu'aient été lues les pressions systolique et diastolique recherchées d'un patient en cours d'examen.

Selon l'invention, la valve ou soupape 28 et son mécanisme d'actionnement sont logés dans le corps 20 de l'appareil, lequel est percé d'un forage 21 terminé par un embout 22 de fixation du tube souple 11. Le forage 21, qui communique par un canal 23 avec le manomètre 14 communique également à son extrémité opposée à l'embout 22 avec une chambre 24 limitée par une paroi 25 du corps 20 de l'appareil et la face 26 en regard, mais qui en est distante, d'un bloc 27 renfermant la valve ou soupape 28 et son mécanisme d'actionnement 29.

De façon plus précise, le bloc 27 sur lequel est fixé la poire 12 et qui est positionné par rapport au corps 20 à l'aide d'une nervure 30 de sa face 26 et d'une gorge 31 du corps est percé de quatre forages 32, 33, 34 et 35. Le premier, 32, débouche sur la face antérieure du sphygmomanomètre au voisinage de l'organe de commande ou bouton 17 et est muni à son autre extrémité débouchant dans le volume intérieur à la poire 17 d'un clapet 36 à lamelle d'élastomère 37, tandis que le second, 33, qui débouche également à une de ses extrémités dans le volume intérieur à la poire 12 débouche à son autre extrémité sur la face 26 où il est muni, lui aussi, d'un clapet 38 à lamelle d'élastomère 39. Le troisième forage, 34, débouche d'une part sur la face 26 du bloc 27 et, d'autre part, dans le quatrième forage 35, qui est un forage borgne de réception de la soupape ou valve 28 et de son mécanisme d'actionnement 29.

Comme bien montré sur la figure 11, la valve 28 est constituée par un siège fixe 38 d'un manchon en matière plastique, par exemple en Téflon (Marque Déposée de la Société Du Pont De Nemours) dont l'alésage axial 39 est tronconique suivant un angle et par une portée tronconique 40 d'angle correspondant d'un organe mobile ou tige 41. A la portée 40 de l'organe mobile ou tige 41 de la valve est adjacente, d'une part, une partie moletée 42 se prolongeant par une tête 43 et, d'autre part, une queue 44 d'appui et de centrage d'un ressort à boudin 45 tendant à constamment appliquer la tige 41 contre le siège de soupape 38.

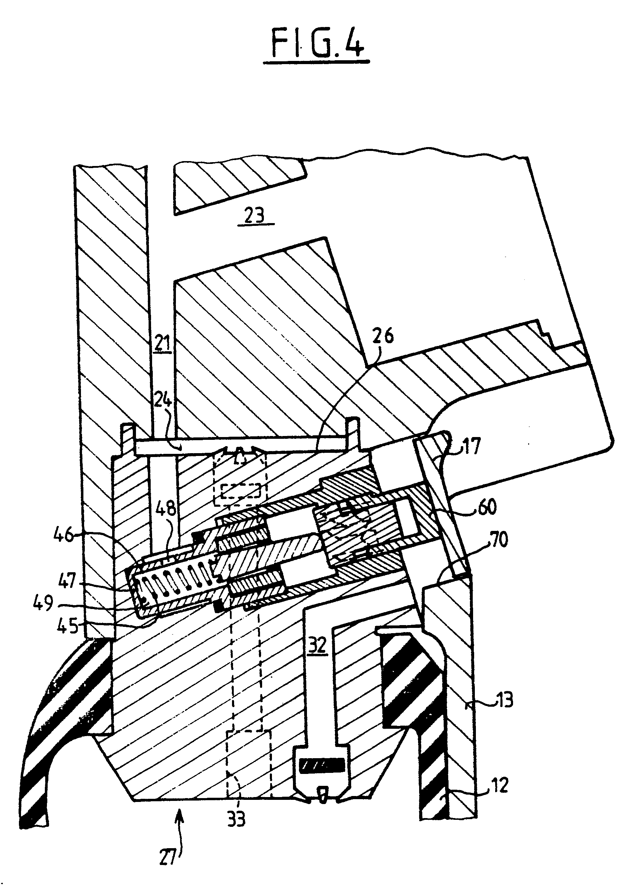

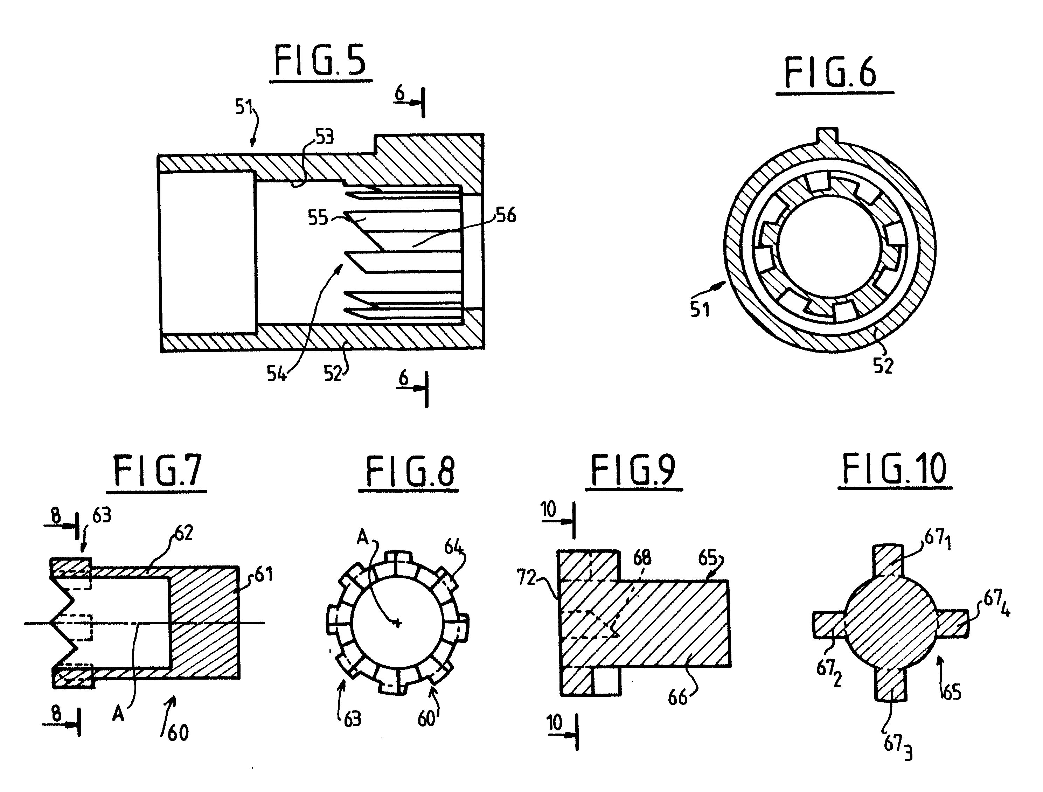

Comme indiqué ci-dessus, cette dernière est logée dans le forage 35 du bloc 27 de l'appareil par l'intermédiaire d'une douille 46 dont le fond 47 sert d'appui au ressort 45 et qui est percée sur sa paroi latérale d'une lumière 48 de mise en communication du forage 34 et du volume 49 intérieur à la douille 46, figure 4. Dans le forage 35 où la douille 46 est montée avec interposition d'un joint d'étanchéité 50, est également monté le mécanisme d'actionnement de la soupape 28. Celui-ci comprend une première pièce 51 qui, comme bien visible sur les figures 5 et 6, est constituée par un fourreau 52 dont la surface interne 53 est conformée, à son extrémité la plus proche du bouton 17 suivant un jeu de rampes 54 de grande et de petite hauteur, 55 et 56, respectivement. Dans le fourreau 52 est montée à coulissement une deuxième partie 60 du mécanisme d'actionnement, figures 7 et 8, qui constitue le poussoir dudit mécanisme et qui est en forme générale de cuvette à fond plein 61 et paroi latérale cylindrique 62 dont l'extrémité libre porte une denture 63 à huit dents 64 régulièrement réparties angulairement autour de l'axe A de ladite pièce. Dans celle-ci est montée la troisième partie 65 du mécanisme d'actionnement, figures 9 et 10, conformée suivant un doigt cylindrique 66, -de diamètre externe correspondant au diamètre interne de la paroi 62 de la pièce 60 dans laquelle il est logé-, et dont l'extrémité distante du fond 61 du poussoir 60 porte quatre saillies radiales $67_1$, $67_2$, $67_3$ et $67_4$ dont les faces frontales sont taillées en biseau comme montré en 68 sur la figure 9 et propres à coopérer avec la denture 63 du poussoir 60.

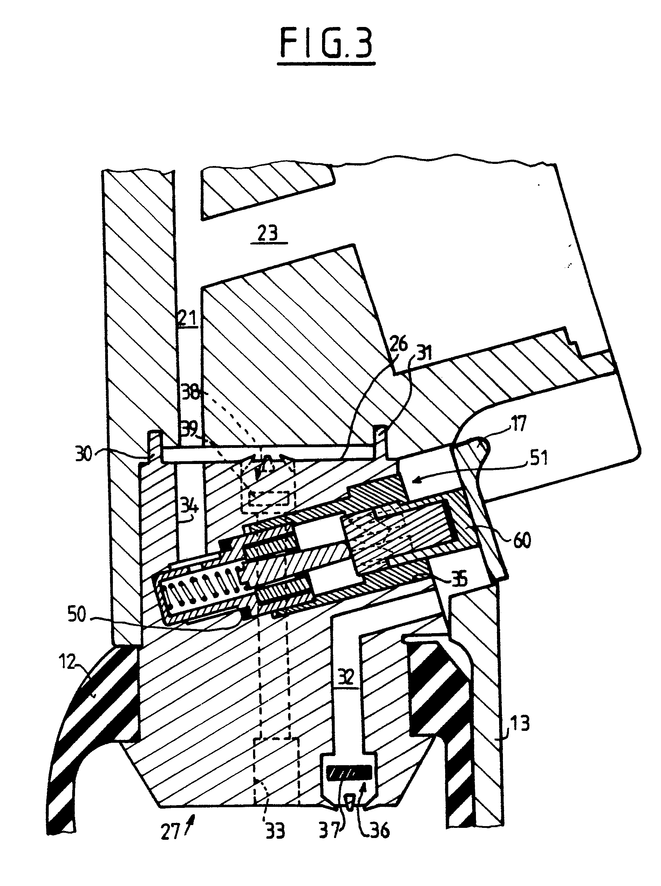

Ce dernier peut être actionné directement par l'utilisateur du sphygmomanomètre ou, comme montré sur les figures 3 et 4, être adjacent au bouton 17 apparent sur la face frontale antérieure de l'appareil et monté à coulissement dans un évidement correspondant 70 du corps 20.

Le mécanisme à dentures qui vient d'être décrit et qui est en contact par la face frontale 72 de la pièce 65 sur la tête 43 de l'organe mobile de la valve 28 permet, par appui direct sur le poussoir 60 ou par l'intermédiaire du bouton 17 d'écarter la portée 40 de l'organe mobile 41 du siège fixe 38 de la valve, comme montré sur la figure 4, et de bloquer ladite valve dans sa condition de pleine ouverture en fin d'appui sur le bouton-poussoir 17-60 tandis qu'un nouvel appui sur cet ensemble, par rotation d'un huitième de tour de la pièce 65, permet un déblocage et une retour du dispositif dans sa condition initiale.

Le fonctionnement du sphygmomanomètre selon l'invention est le suivant. Après mise en place du brassard de mesure, l'utilisateur de l'appareil agit sur la poire 12 de manière usuelle pour gonfler le brassard par un circuit pneumatique comprenant le forage 32 et le clapet 36, pour l'introduction d'air dans la poire, et le forage 33 et le clapet 38 pour l'éjection de l'air préablement aspiré dans le tube 11 via la chambre 24 et le forage 21.

Pour la réduction progressive de la pression du brassard l'utilisateur de l'appareil appuie alors sur le bouton-poussoir 17-60 dont la condition initiale est celle montrée sur la figure 3, en laquelle la valve 28 est fermée. Cet appui commande le coulissement de la tige 41, contre l'action du ressort 45, et l'air enfermé dans le brassard s'échappe par un circuit comprenant le forage 21, la chambre 24, le forage 34, la lumière 48, l'espace 49, la lumière de fuite entre le siège 38 et la portée 40. Au fur et à mesure que la pression décroît dans le brassard, la valeur correspondante s'affiche sur le manomètre proprement dit 14 pneumatiquement relié par le canal 23 au forage 21. Après lecture des pressions systolique et diastolique, l'utilisateur de l'appareil en poursuivant son appui sur le bouton-poussoir 17-60 provoque le blocage en condition ouverte de la soupape par coopération avec les rampes 55 de grande hauteur des dents 64 du poussoir 60. Dans cette condition, le brassard est rapidement et complètement mis à l'air libre et peut ainsi être retiré du bras ou autre membre du patient qui le portait.

Pour ramener l'appareil dans sa condition initiale, permettant une autre mesure, un appui sur le bouton-poussoir 17-60 fait échapper la denture 63 aux rampes de la pièce 51 et, après une rotation d'un huitième de tour de la pièce 65, celle-ci et le poussoir 60 sont ramenés sous l'action du ressort 45 et par l'intermédiaire de la tige 41, dans leur condition initiale.

**Revendications**

1. Sphygmomanomètre comprenant, réunis en un ensemble unitaire, une poire (12) de mise en pression d'un brassard de mesure, un manomètre (14) de lecture proprement dit de la pression artérielle, une valve ou soupape (28) de mise à l'air libre du brassard et des moyens de commande pour l'ouverture progressive de ladite valve ou soupape pour réduire graduellement la pression du brassard gonflé en vue de la détermination des pressions systolique et diastolique, d'une part, et de maintien de la valve (28) dans sa condition de pleine ouverture pour la mise à l'air libre complète et rapide du brassard, d'autre part, caractérisé en ce que lesdits moyens de commande sont réalisés sous forme d'un bouton (17) monté à coulissement et auquel est associé un mécanisme de poussoir à dentures (51, 60, 65) organisé de manière telle que le blocage de ladite valve (28) dans sa condition de pleine ouverture pour la mise à l'air libre rapide du brassard est obtenu automatiquement après une course d'enfoncement prédéterminée du bouton-poussoir, un nouvel appui sur ce dernier provoquant le déblocage de la valve (28) et sa fermeture sous l'action de moyens élastiques (45) préalablement mis sous tension, ainsi que le retour du bouton-poussoir dans sa condition initiale pour une nouvelle mesure de pression.

2. Sphygmomanomètre selon la revendication 1, caractérisé en ce que le mécanisme de poussoir à dentures (51, 60, 65) associé au bouton (17) est du type à rampes frontales (54) et est disposé dans le corps (20) entre le bouton-poussoir (17-60) et l'organe mobile (41) de la valve ou soupape (28).

3. Sphygmomanomètre selon la revendication 2, caractérisé en ce que la valve (28) est constituée par un siège fixe (38) ménagé par l'alésage axial tronconique (39) d'un manchon et par l'organe mobile conformé suivant une tige (41) montée à coulissement dans le manchon, ladite tige (41) présentant une portée tronconique (40) sur une partie de sa longueur, entre une queue (44) de centrage des moyens élastiques de rappel (45) dudit organe et une tête d'appui (43) du bouton-poussoir (17-60).

4. Sphygmomanomètre selon l'une quelconque des revendications précédentes, caractérisé en ce que la valve ou soupape (28) et ses moyens de commande et d'actionnement (51-60-65) de même que les clapets (36, 38) permettant le gonflage du brassard à l'aide de la poire (12) de mise en pression sont logés dans un bloc (27) propre à être fixé au corps (20) de l'appareil.

## FIG.1

## FIG.11

EP 0 363 229 A1

FIG.2

# FIG.3

# FIG.4

FIG.6

FIG.5

FIG.10

FIG.9

FIG.8

FIG.7

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | DE-U-8 629 328  (B. SPEIDEL)<br>* page 3, ligne 26 - page 4, ligne 36;<br>page 6, ligne 10 - page 10, ligne 7;<br>figure 1 * | 1 | A 61 B   5/02 |
| A | | 2-4 | |
| | --- | | |
| A | US-A-4 690 171  (C.F. JOHNSTON)<br>* abstract; figures 2,3,4 * | 1 | |
| | --- | | |
| A | DE-U-8 708 985  (R. RIESTER GMBH)<br>* page 5, ligne 15 - page 7, ligne 18;<br>figures 1,2,3 * | 1 | |
| | --- | | |
| Y | EP-A-0 079 617  (FIRMA R. KALLMEYER)<br>* page 17, ligne 20 - page 18, ligne<br>29; page 21, ligne 13 - page 23, ligne<br>18; figures 1-3 * | 1 | |
| A | ----- | 4 | |

|  |
|---|
| **DOMAINES TECHNIQUES RECHERCHES (Int. Cl.4)** |
| A 61 B   5/00 |

Le présent rapport a été établi pour toutes les revendications

| Lieu de la recherche | Date d'achèvement de la recherche | Examinateur |
|---|---|---|
| BERLIN | 27-10-1989 | WEIHS J.A. |